# EUROPÄISCHE PATENTSCHRIFT

(11) **EP 3 849 435 B1**
(45) Veröffentlichungstag und Bekanntmachung des Hinweises auf die Patenterteilung: **07.02.2024**
(21) Anmeldenummer: 19724401.5
(22) Anmeldetag: 09.05.2019
(51) Int. Cl.: A61B 17/16, A61B 17/29, A61B 17/00

(54) **CHIRURGISCHES INSTRUMENT**
SURGICAL INSTRUMENT
INSTRUMENT CHIRURGICAL

(30) Priorität: 12.09.2018 DE 102018122237
(43) Veröffentlichungstag der Anmeldung: 21.07.2021
(73) Patentinhaber: Morpheus AG, 78549 Spaichingen (DE)
(72) Erfinder: RACK, Timo, 78604 Rietheim-Weilheim (DE); BLESSING, Heiko, 78591 Durchhausen (DE)
(74) Vertreter: Mertzlufft-Paufler, Cornelius
(86) Internationale Anmeldenummer: PCT/EP2019/061980
(87) Internationale Veröffentlichungsnummer: WO 2020/052816

(56) Entgegenhaltungen:
- US-A- 5 052 243
- US-A- 5 499 548
- US-A1- 2010 030 029
- US-A1- 2018 000 514

## Beschreibung

Die Erfindung betrifft ein chirurgisches Instrument mit einem Griffbereich, der ein in Gebrauchsstellung feststehendes erstes Griffteil und ein relativ zum ersten Griffteil in einer Verstellungsrichtung bewegbares zweites Griffteil aufweist. Insbesondere kann das chirurgische Instrument zur Gewebeabtrennung eingerichtet sein, wie beispielsweise als Gewebestanze. Bekannte Gewebestanzen sind hierbei zum Beispiel sogenannte Kerrison oder Rongeur Stanzen.

US 2018/0000514 A1 betrifft ein Schaftinstrument für chirurgische Zwecke, mit einem Schaftteil, der an einem Ende fest mit einem Schaftgriff verbunden ist, und mit einem Schiebeteil, das relativ zum Schaftteil in dessen Längsrichtung verschiebbar gelagert ist und an einem Ende mit einem vorgespannten Handgriff derart wirkverbunden ist, dass durch Betätigung des Handgriffs das Schiebeteil gegen die Vorspannung verschiebbar ist. Der Handgriff weist einen Kupplungsabschnitt auf, in welchem zwei Hebelarme des Handgriffs in Abstand voneinander miteinander gekoppelt sind.

Ähnliche chirurgische Instrumente sind aus US 2010/0030029 A1, US 5,052,243 A und US 5,499,548 A bekannt.

Typische chirurgische Instrumente der eingangs genannten Art weisen den Nachteil auf, dass ein Benutzter, der beispielsweise ein Operateur sein kann, bei zeitintensiven Arbeiten mit dem chirurgischen Instrument relativ schnell ermüdet. Der Benutzer muss während der Arbeit mit dem chirurgischen Instrument den Griffbereich mittels seiner Handkraft mehrfach betätigen, was nach einer Weile dazu führen kann, dass ihm der Arm und die Hände schmerzen, da die Muskulatur ermüdet. Dies beeinträchtigt die Genauigkeit seiner Arbeit. Beispielsweise werden die Instrumente dazu eingesetzt, um an der Wirbelsäule Knochenmaterial oder Bandscheibenmaterial in der Nähe von Nerven zu entfernen, so dass ein besonders genaues Betätigen des Instruments wichtig ist, um Verletzungen an den Nerven zu vermeiden. Häufig versuchen sich die Benutzer auch dadurch zu helfen, dass sie die Hand zur Betätigung des chirurgischen Instruments wechseln.

Es besteht somit die Aufgabe, ein chirurgisches Instrument der eingangs genannten Art zu schaffen, bei welchem die genannten Nachteile ausgeräumt sind und welches einen ergonomischen Griffbereich aufweist, der einen Benutzer durch die Benutzung des chirurgischen Instruments weniger schnell ermüden lässt.

Diese Aufgabe wird erfindungsgemäß durch die Merkmale des unabhängigen Anspruchs gelöst. Insbesondere wird zur Lösung der Aufgabe ein chirurgisches Instrument der eingangs genannten Art vorgeschlagen, dass sich dadurch kennzeichnet, dass am zweiten Griffteil wenigstens zwei versetzt zueinander angeordnete Gelenkpunkte ausgebildet sind, durch welche eine Verstellungsrichtung des zweiten Griffteils relativ zum ersten Griffteil definiert ist. Durch die erfindungsgemäße Ausgestaltung ist es möglich, dass ein Benutzer länger mit dem chirurgischen Instrument arbeiten kann, da er damit weniger schnell erschöpft ist als bei vorbekannten Instrumenten. Dies ist darauf zurückzuführen, dass der bewegliche Griffteil nicht um einen einzigen Gelenkpunkt oder Drehpunkt verstellt wird, sondern um zwei, versetzt zu einander angeordnete Gelenkpunkte. Somit ist es möglich, dass durch eine Betätigung des zweiten Griffteils das zweite Griffteil in einer Art Parallelbewegung oder Ellipsenbewegung oder Schwenkbewegung auf das erste Griffteil zubewegt werden kann und nicht wie üblich in einer Kreisbewegung um einen einzigen Gelenkpunkt. Bei der Bewegung entlang einer Kreisbahn liegt bei den meisten chirurgischen Instrumenten der eingangs genannten Art der kleine Finger der Hand am längsten, durch das bewegliche Griffteil ausgebildeten Hebel an. Diese vorbekannten Instrumente weisen meist einen V-förmigen Griffbereich auf. Das hat zu Folge, dass mittels des kleinen Fingers der größte Kraftaufwand erbracht werden muss, um das bewegliche Griffteil zu verstellen. Dies kann schließlich zu einer schnelleren Ermüdung der Muskulatur des Benutzers führen. Allerdings kann die Geometrie des chirurgischen Instruments der eingangs genannten Art nicht grundsätzlich abgeändert werden, so dass der kleine Finger auch weiterhin am nächsten zum freien Ende des beweglichen Griffteils angelegt werden muss. Durch die über wenigstens eine Teilstrecke des Betätigungswegs parallele oder nahezu parallele Ausrichtung der beiden Griffteile und/oder die elliptische Relativbewegung zwischen den Griffteilen beim erfindungsgemäßen chirurgischen Instrument ist eine deutlich gleichmäßigere Übertragung der Handkraft möglich. Die Verstellungsrichtung kann somit zumindest über eine erste Hälfte eines Betätigungsweges geradliniger, insbesondere nahezu geradlinig, oder auf einer elliptischen Bahn verlaufen.

Ein Verhältnis zwischen einer ersten Strecke zwischen dem ersten und dem zweiten Griffteil und einer zweiten Strecke zwischen dem ersten und dem zweiten Griffteil kann somit während einer Bewegung des zweiten Griffteils in Richtung des ersten Griffteils wenigstens über die erste Hälfte eines Betätigungsweges konstant oder nahezu konstant sein. Dabei können die erste Strecke und die zweite Strecke parallel zueinander verlaufen. Alternativ oder ergänzend können die erste Strecke und die zweite Strecke parallel zu einer Schaftaufnahme und/oder einer Gleitschiene des chirurgischen Instruments verlaufen. Alternativ oder ergänzend können ein Abstand zwischen der ersten Strecke und der Schaftaufnahme und ein Abstand zwischen der zweiten Strecke und der Schaftaufnahme unterschiedlich groß sein. Alternativ oder ergänzend können sich die Strecken jeweils zwischen einer Handanlagefläche am ersten Griffteil und einer Handanlagefläche am zweiten Griffteil erstrecken.

Nachfolgend werden vorteilhafte Ausgestaltungen der Erfindung beschrieben, die allein oder in Kombination mit den Merkmalen anderer Ausgestaltungen optional zusammen mit den Merkmalen nach Anspruch 1 kombiniert werden können.

Die Verstellungsrichtung des zweiten Griffteils ist durch wenigstens zwei Führungselemente festgelegt, wobei die beiden Führungselemente jeweils über einen Gelenkpunkt mit dem ersten Griffteil und über jeweils einen Gelenkpunkt mit dem zweiten Griffteil verbunden sind, wobei deren Gelenkpunkte am ersten Griffteil versetzt zueinander angeordnet sind, und wobei deren Gelenkpunkte am zweiten Griffteil versetzt zueinander angeordnet sind. Bei den Gelenkpunkten kann es sich beispielsweise um gelagerte Drehachsen handeln. Insbesondere um lediglich in einem Freiheitsgrad verstellbare und/oder in einem Drehlager gelagerte Drehachsen. Dies hat den Vorteil, dass eine besonders einfache Führung des beweglichen zweiten Griffteils ausgebildet ist, die aus möglichst wenigen Teilen besteht. Die Führungselemente können beispielsweise als Führungshebel ausgebildet sein. Beispielsweise kann/können wenigstens einer der Führungshebel oder alle Führungshebel steif, also insbesondere unflexibel, ausgebildet sein, um eine besonders gute Führung zu ermöglichen. Ein Gelenkpunkt im Sinne der Anmeldung kann beispielsweise eine in einem Drehlager geführte Drehachse sein. Die Drehachse kann beispielsweise am Griffteil und/oder am Führungselement und/oder am Antriebselement ausgebildet sein.

Um einen Hub des chirurgischen Instruments noch weiter erhöhen zu können, ohne den Abstand zwischen den beiden Griffteilen in einer Ruhestellung erweitern zu müssen, weist das chirurgische Instrument ein Antriebselement auf, das über einen Lagerpunkt mit dem zweiten Griffteil verbunden ist und das über einen Lagerpunkt mit dem ersten Griffteil verbunden ist. Bei wenigstens einem Lagerpunkt oder bei beiden Lagerpunkten kann es sich zum Beispiel um einen Gelenkpunkt handeln. Besonders vorteilhaft kann ein wandernder Gelenkpunkt sein, der beispielsweise innerhalb eines Führungsabschnitts wandern kann, wenn das zweite Griffteil von einer Offenstellung in eine Schließstellung bewegt wird und umgekehrt. Der Führungsabschnitt kann dabei beispielsweise als ein Langloch, insbesondere als ein gerades oder ein gebogenes Langloch, ausgestaltet sein, in welchem eine in wenigstens zwei Freiheitsgraden bewegliche, entlang des Führungsabschnitts wandernde Drehachse geführt ist. Beispielsweise kann/können der Lagerpunkt oder die Lagerpunkte des Antriebselements am ersten Griffteil und/oder am zweiten Griffteil zu einem Gelenkpunkt, beispielsweise dem bereits genannten Gelenkpunkt, zumindest eines der Führungselemente am ersten Griffteil und/oder am zweiten Griffteil versetzt angeordnet sein. Das Antriebselement kann beispielsweise als Antriebshebel ausgebildet sein. Um eine besonders gute Kraftübertragung durch den Antriebshebel erreichen zu können, kann dieser steif, also insbesondere unflexibel ausgebildet sein.

Um einen möglichst großen Hub des chirurgischen Instruments bei kleinstmöglichem Abstand zwischen den Griffteilen einrichten zu können, kann gemäß einer weiteren Ausgestaltung vorgesehen sein, dass wenigstens eines der Führungselemente und/oder ein Antriebselement, beispielsweise das bereits zuvor genannte Antriebselement, eine Biegung aufweist/aufweisen. Dabei kann es besonders vorteilhaft sein, wenn das wenigstens eine Führungselement und/oder das Antriebselement in Richtung des ersten und/oder des zweiten Griffteils geneigt ist/sind.

Gemäß einer Weiterbildung kann das chirurgische Instrument einen Schaft mit einer Führungsschiene und einer relativ zur Führungsschiene verstellbaren Gleitschiene aufweisen, wobei bei einer Betätigung des zweiten Griffteils eine Kraftübertragung von wenigstens einem Führungselement und/oder von einem Antriebselement, beispielsweise dem bereits zuvor genannten Antriebselement, auf die Gleitschiene des Schaftes erfolgt. Insbesondere kann der Schaft ein Schneid- und/oder Stanzwerkzeug aufweisen. Ein Hub des chirurgischen Instruments kann sich somit auf einen maximalen relativen Verstellungsweg zwischen der Gleit- und der Führungsschiene beziehen.

Um eine möglichst geradlinige oder genau geradlinige Verstellung eines Teils eines in die Schaftaufnahme eingesetzten Schaftes erreichen zu können, kann es vorteilhaft sein, wenn das chirurgische Instrument eine Ausgleichsvorrichtung aufweist, in welche ein Eingriffszapfen eingreift und eine Beaufschlagungsfläche der Ausgleichsvorrichtung kontaktiert, wobei bei einer Betätigung des zweiten Griffteils eine Kreisbewegung des Eingriffszapfens in eine geradlinige Bewegung der Ausgleichsvorrichtung umgesetzt wird oder umsetzbar ist. Beispielsweise derart, dass ein Kontaktpunkt des Eingriffszapfens an der Beaufschlagungsfläche in einer Richtung quer oder senkrecht zur geradlinigen Bewegung entlang der Beaufschlagungsfläche wandert. Die Ausgleichsvorrichtung kann beispielsweise an einer Gleitschiene des chirurgischen Instruments ausgebildet sein. Der Eingriffszapfen kann beispielsweise am Antriebselement ausgebildet sein.

Um eine Verstellung der Ausgleichsvorrichtung entlang einer geradlinigen Verschiebungsrichtung festzulegen, kann die Ausgleichsvorrichtung fest mit einer oder der Gleitschiene verbunden sein. Fest verbunden kann in diesem Zusammenhang insbesondere in einem Öffnungswinkel unverstellbar bedeuten. Insbesondere kann die Ausgleichsvorrichtung derart ausgestaltet sein, dass dabei ein Winkel zwischen der Beaufschlagungsfläche und der Gleitschiene während einer Betätigung des chirurgischen Instruments unverändert bleibt.

Dies erlaubt eine besonders effiziente Handkraftübertragung, da die Handkraft nahezu vollständig in eine geradlinige Verstellungskraft umsetzbar ist oder während eines Betätigungsvorgangs umgesetzt wird.

Gemäß einer weiteren Ausgestaltung kann am ersten Griffteil und/oder am zweiten Griffteil eine Eintauchausnehmung zum Eintauchen wenigstens eines Führungselements in den ersten und/oder den zweiten Griffteil ausgebildet sein. Somit ist es möglich, den Betätigungsweg zwischen den Griffteilen möglichst groß auszugestalten, ohne den Abstand zwischen den Griffteilen erhöhen zu müssen. In Schließstellung dringen die Führungselemente und/oder das Antriebselement in die Eintauchausnehmung/en ein, so dass die Griffteile möglichst nahe aufeinander zubewegbar sind. Dies hat den Vorteil, dass der Hub des chirurgischen Instruments noch größer ausgestaltet werden kann.

Um ein Rückstellen der Griffteile in die Offenstellung mittels Handkraft zu vermeiden, kann das chirurgische Instrument gemäß einer weiteren Ausgestaltung ein Rückstellelement, beispielsweise eine Rückstellfeder, aufweisen, dessen Rückstellkraft einer Relativbewegung des ersten Griffteils hin zum zweiten Griffteil entgegenwirkt. Um eine sichere Führung und Lagerung des Rückstellelements auszubilden, kann an einem der Führungselemente eine Rückstellelementaufnahme ausgebildet sein, in welche das Rückstellelement eingesetzt ist. Beispielsweise kann das Rückstellelement als eine Schenkelfeder ausgebildet sein, deren Drehzentrum an einem Gelenkpunkt eines der Führungselemente am ersten Griffteil ausgebildet ist und/oder mit diesem überlappt.

Um den Abstand zwischen den beiden Griffteilen in Offenstellung und/oder in Schließstellung möglichst klein halten zu können, können an den Führungselementen jeweils zwei, insbesondere unterschiedliche Ausrichtungen aufweisende Anlageflächen ausgebildet sein, wobei ein erstes Paar an Anlageflächen der beiden Führungselemente in einer maximalen Offenstellung aneinander anliegen und ein zweites Paar an Anlageflächen in einer maximalen Schließstellung aneinander anliegen.

Gemäß einer Ausgestaltung kann ein Lagerpunkt, beispielsweise der bereits zuvor genannte Lagerpunkt, eines Antriebselements, beispielsweise des bereits zuvor genannten Antriebselements, am ersten Griffteil weiter entfernt von einer Schaftaufnahme, beispielsweise der bereits zuvor genannten Schaftaufnahme, und/oder von einem Schaft, beispielsweise dem bereits zuvor genannten Schaft, angeordnet sein als die Gelenkpunkte der Führungselemente am ersten Griffteil. Somit ist ein besonders großer Hub einrichtbar.

Um den Platzbedarf eines Antriebselements, beispielsweise des bereits zuvor genannten Antriebselements, zu verringern, kann das Antriebselement wenigstens teilweise in einer Aussparung in einem Führungselement angeordnet sein. Somit kann das Antriebselement an der Aussparung wenigstens teilweise in das Führungselement eindringen. Das Führungselement mit der Aussparung kann das Antriebselement lateral zumindest teilweise und/oder beidseitig flankieren.

Um das Greifen und Betätigen des chirurgischen Instruments insbesondere für eine Person mit relativ kleinen Händen zu erleichtern, kann ein freies Ende des zweiten Griffteils in Ruheposition in Richtung des ersten Griffteils geneigt sein. Alternativ oder ergänzend kann das freie Ende des zweiten Griffteils in Ruhestellung näher am ersten Griffteil liegen als ein Griffmittelpunkt des zweiten Griffteils.

Um das chirurgische Instrument wiederverwenden zu können, kann es vorteilhaft sein, wenn das Instrument aus einem autoklavierbaren Material oder autoklavierbaren Materialien hergestellt ist. Somit ist eine Sterilisation des Instruments durch Temperaturen von über 120°C möglich. Beispielsweise kann das chirurgische Instrument aus einem Metall oder mehreren Metallen hergestellt sein.

Um den Hub des chirurgischen Instruments noch weiter erhöhen zu können, ohne dass der Abstand zwischen den beiden Griffteilen erweitert werden muss, kann es gemäß einer weiteren Ausgestaltung vorgesehen sein, dass an wenigstens einem Führungselement eine Drehachsenaussparung ausgebildet ist. Wird das zweite Griffteil auf das erste Griffteil zubewegt, so kann das Führungsteil mit der Drehachsenaussparung in Schließstellung besonders nahe an die Drehachse des Antriebselements am zweiten Griffteil herangeführt werden, da die Drehachse des Antriebselements in die Drehachsenaussparung eindringt.

Die Erfindung betrifft zudem eine Verwendung eines chirurgischen Instruments, wie es hierin beschrieben und beansprucht ist, zur Bewegung eines Schneide- und/oder Stanzwerkzeuges. Somit ist eine besonders kraftschonende Handhabung des chirurgischen Instruments möglich, wie zuvor bereits ausführlich erläutert wurde.

Die Erfindung wird nun anhand eines Ausführungsbeispiels näher beschrieben, ist jedoch nicht auf dieses Ausführungsbeispiel beschränkt. Weitere Ausführungsbeispiele ergeben sich durch die Kombination der Merkmale einzelner oder mehrerer Ansprüche untereinander und/oder mit einzelnen oder mehreren Merkmalen des Ausführungsbeispiels.

Es zeigt:
- Fig. 1: eine Seitenansicht einer möglichen Ausgestaltungsform des erfindungsgemäßen chirurgischen Instruments, wobei das Instrument in einer Offenstellung, also in Ruhestellung gezeigt ist,
- Fig. 2: eine perspektivische Darstellung des chirurgischen Instruments aus Fig. 1,
- Fig. 3: eine isometrische Ansicht des chirurgischen Instruments aus den zuvor genannten Figuren, mit teilweise geöffnetem Gehäuse,
- Fig. 4: eine weitere isometrische Ansicht des chirurgischen Instruments aus Fig. 3,
- Fig. 5: eine weitere isometrische Ansicht des chirurgischen Instruments aus Fig. 3 und 4,
- Fig. 6: eine weitere isometrische Ansicht des chirurgischen Instruments aus den zuvor genannten Figuren,
- Fig. 7: eine Schnittansicht des chirurgischen Instruments aus den zuvor genannten Figuren,
- Fig. 8: eine Seitenansicht eines zum Teil transparent dargestellten chirurgischen Instruments aus den zuvor genannten Figuren.

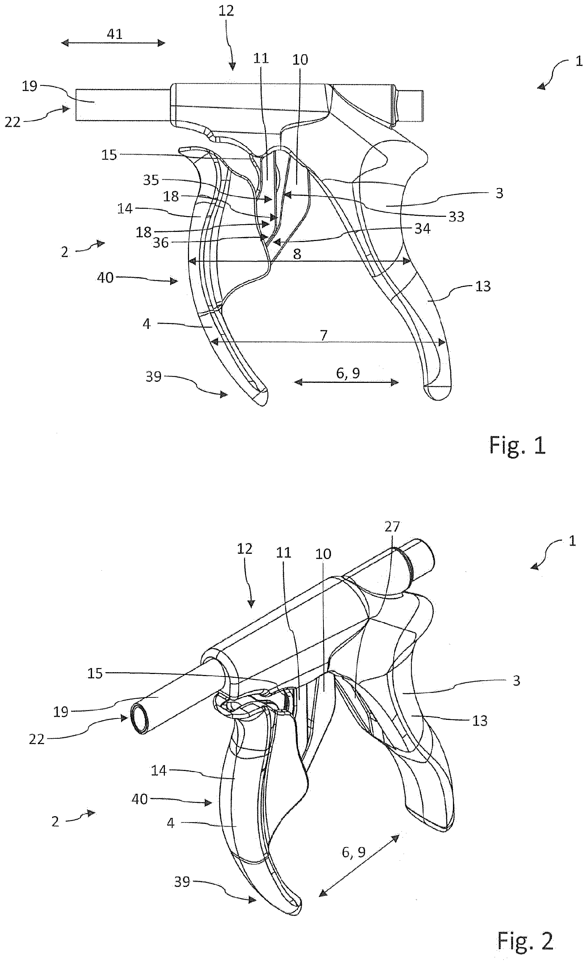

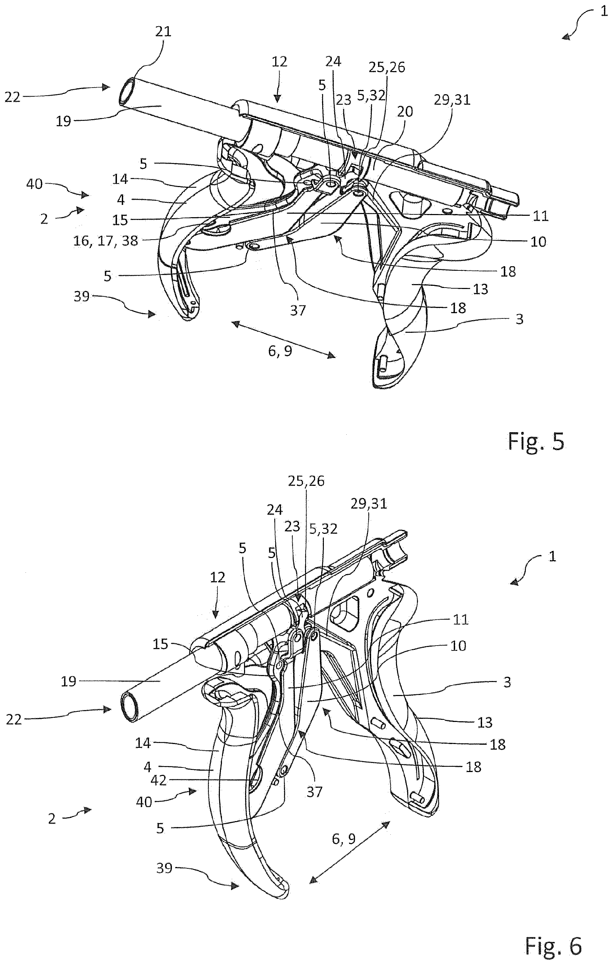

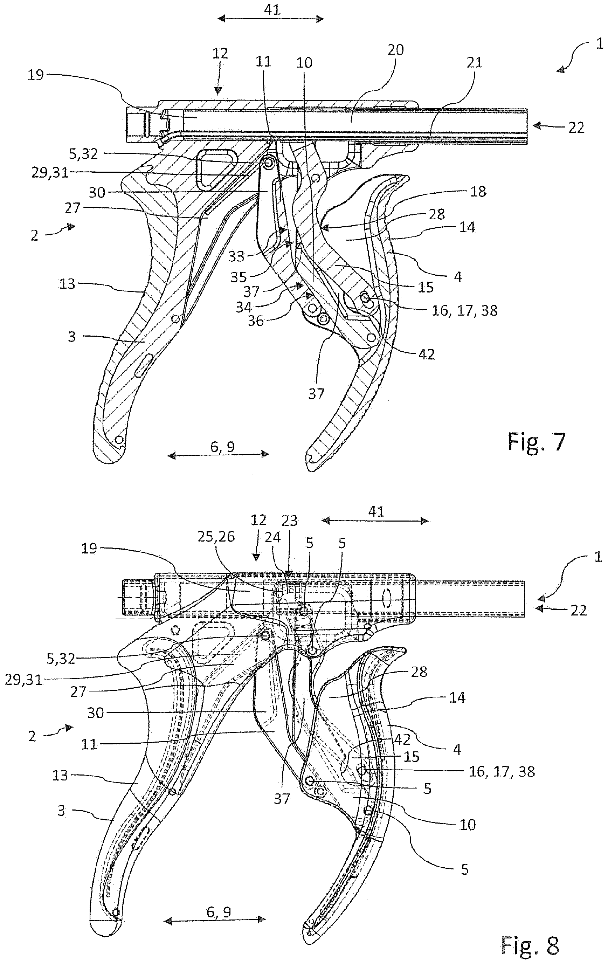

In den Figuren 1 bis 8 ist eine mögliche Ausführungsform eines chirurgischen Instruments dargestellt, das im Ganzen als 1 bezeichnet ist.

Das chirurgische Instrument 1 weist einen Griffbereich 2 auf, der es einem Benutzer ermöglicht, das chirurgische Instrument 1 zu halten und zu bedienen.

Der Griffbereich 2 weist ein erstes Griffteil 3 und ein zweites Griffteil 4 auf. Das erste Griffteil 3 ist als feststehendes, also in Gebrauch vom Nutzer festgehaltenes Griffteil ausgestaltet. Das zweite Griffteil 4 ist beweglich gelagert, so dass es in Gebrauchsstellung relativ zum ersten Griffteil 3 entlang einer Verstellungsrichtung durch den Benutzer verstellbar ist. Bei einer Betätigung des zweiten Griffteils während des Gebrauchs des chirurgischen Instruments 1 kommt es somit zu einer Bewegung des zweiten Griffteils 4 in Richtung des ersten Griffteils 3.

Am zweiten Griffteil 4 sind wenigstens zwei, versetzt zueinander angeordnete Gelenkpunkte 5, 17 ausgebildet, durch welche die Verstellungsrichtung 6 des zweiten Griffteils 4 relativ zum ersten Griffteil 3 festgelegt ist, da das zweite Griffteil 4 um beide Gelenkpunkte 5 bewegbar angeordnet ist.

Durch die Ausgestaltung der beiden Gelenkpunkte 5, 17 am zweiten Griffteil 4 ist es möglich, eine reine Drehbewegung des zweiten Griffteils 4 um eine einzige Drehachse zu vermeiden. Somit ist eine bessere Handkraftübertragung möglich. Das zweite Griffteil 4 wird durch eine Betätigung vielmehr durch eine Schwenkung um beide Gelenkpunkte 5, 17 auf das erste Gelenkteil 3 zubewegt.

Die zuvor genannte Verstellungsrichtung 6 des zweiten Griffteils 4 in Richtung des ersten Griffteils 3 ist durch wenigstens zwei Führungselemente 10, 11 festgelegt. Das erste Führungselement 10 ist über einen Gelenkpunkt 5 mit dem ersten Griffteil 3 und über einen Gelenkpunkt 5 mit dem zweiten Griffteil 4 verbunden. Das zweite Führungselement 11 ist über einen Gelenkpunkt 5 mit dem ersten Griffteil 3 und über einen Gelenkpunkt 5 mit dem zweiten Griffteil 4 verbunden.

Der Gelenkpunkt 5 des ersten Führungselements 10 und der Gelenkpunkt 5 des zweiten Führungselements 11 jeweils am ersten Griffteil 3 sind versetzt zueinander angeordnet. Der Gelenkpunkt 5 des ersten Führungselements 10 und der Gelenkpunkt 5 des zweiten Führungselements 11 jeweils am zweiten Griffteil 4 sind ebenfalls versetzt zueinander angeordnet.

Bei den Gelenkpunkten 5 kann es sich beispielsweise um in einem Drehlager geführte Rotationsachsen handeln.

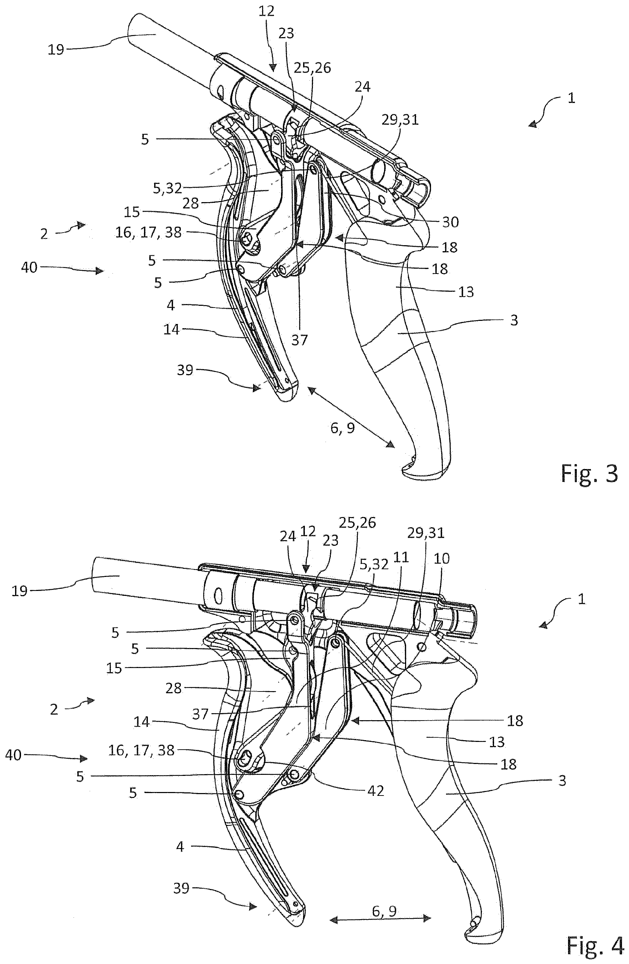

Das chirurgische Instrument 1 gemäß den Figuren 1 bis 8 weist weiter ein Antriebselement 15 auf, das über einen Lagerpunkt 16 mit dem zweiten Griffteil 4 verbunden ist. Der Lagerpunkt 16 kann, wie in den Figuren 3 bis 8 dargestellt ist, beispielsweise als ein Gelenkpunkt 17 mit wandernder Drehachse ausgebildet sein. Die Drehachse kann hierbei beispielsweise in einem als Langloch 38 ausgestalteten Führungsabschnitt angeordnet sein. Das zweite Ende des Antriebselements 15 ist mit dem ersten Griffteil 3 über einen Gelenkpunkt 5 verbunden.

Es sind jedoch auch Ausgestaltungen denkbar, bei welchen das Antriebselement 15 über zwei Lagerpunkte 16, beispielsweise mit jeweils wandernden Drehachsen, gelagert und mit den Griffteilen 3, 4 verbunden ist. Durch die Ausgestaltung einer wandernden Drehachse kann der gesamte Hub des chirurgischen Instruments 1 noch weiter erhöht werden, ohne dass ein Abstand zwischen den beiden Griffteilen 3, 4 erhöht werden muss. Die in dem beispielsweise als Langloch 38 ausgestalteten Führungsabschnitt gelagerte Drehachse kann bei den in den Figuren 3 bis 8 gezeigten chirurgischen Instrument 1 somit von einer ersten, insbesondere oberen Anschlagstellung in Offenstellung des chirurgischen Instruments 1 zu einer zweiten, insbesondere unteren Anschlagstellung bei einer Schließstellung des chirurgischen Instruments 1 wandern.

Der Lagerpunkt 16 des Antriebselements 15 am zweiten Griffteil 4 ist zu den Gelenkpunkten 5 der beiden Führungselemente 10, 11 am zweiten Griffteil 4 versetzt angeordnet. Somit kann mittels des Antriebselements 15 ein deutlich größerer Betätigungsweg 9, der den Hub des chirurgischen Instruments 1 ausmacht, erreicht werden.

Die beiden Führungselemente 10, 11 und das Antriebselement 15 weisen jeweils eine Biegung 18 auf. Wie in den Figuren 1 bis 8 gut zu erkennen ist, kann/können sich die Führungselemente 10, 11 und/oder das Antriebselement 15 aufgrund der Biegung 18 in Richtung des zweiten Griffteils 4 neigen.

Das chirurgische Instrument 1 weist weiter einen Schaft 19 auf, der in der bereits zuvor genannten Schaftaufnahme 12 angeordnet ist. Der Schaft 19 umfasst eine Führungsschiene 20 und eine relativ zur Führungsschiene 20 verstellbare Gleitschiene 21. Die Gleitschiene 21 kann dabei beispielsweise relativ zur feststehenden Führungsschiene 20 beweglich ausgestaltet sein. Die Führungsschiene 20 kann fest mit dem ersten Griffteil 3 verbunden sein, insbesondere derart, dass in Gebrauchsstellung zwischen dem ersten Griffteil 3 und der Führungsschiene 20 keine Relativbewegung möglich ist.

Bei einer Betätigung des zweiten Griffteils 4 ist eine Kraftübertragung des Antriebselements 15 auf die Gleitschiene 21 des Schaftes 19 möglich.

Um ein Abheben der Gleitschiene 21 von der Führungsschiene 20, was durch eine Kreisbewegung des Antriebselements 15 hervorgerufen werden kann, zu vermeiden, weist das chirurgische Instrument 1 eine Ausgleichsvorrichtung 23 auf. Die Ausgleichsvorrichtung 23 umfasst einen Aufnahmeraum mit einer Beaufschlagungsfläche 25. In den Aufnahmeraum der Ausgleichsvorrichtung 23 ist ein Eingriffszapfen 24 eingeführt, der in einem Kontaktpunkt 26 die Beaufschlagungsfläche 25 kontaktiert. Eine Einführrichtung in den Aufnahmeraum der Ausgleichsvorrichtung 23 ist dabei vorzugsweise senkrecht zu einer vorzugsweise geradlinigen Verstellungsbewegung 41 der Ausgleichsvorrichtung 23 ausgerichtet. Durch Betätigung des zweiten Griffteils 4 wandert der Kontaktpunkt 26 des Eingriffszapfens 24 an der Beaufschlagungsfläche 25 entlang schräg oder senkrecht zu der vorzugsweise geradlinigen Verstellungsrichtung 41 der Ausgleichsvorrichtung 23. Dadurch ist eine Kraftübertragung vom Antriebselement 15 auf die Beaufschlagungsfläche 25 möglich. Eine Rotationsbewegung des Eingriffszapfens 24 kann somit in eine lineare Bewegung 41 der Ausgleichsvorrichtung 23 umgesetzt werden.

Die Ausgleichsvorrichtung 23 ist fest mit der Gleitschiene 21 verbunden, so dass die Gleitschiene 21 zusammen mit der Ausgleichsvorrichtung 23 entlang der vorzugsweise geradlinigen Verschiebungsrichtung 41 durch Betätigung des zweiten Griffteils 4 verstellbar ist.

Ein Winkel zwischen der Beaufschlagungsfläche 25 und der Gleitschiene 21 und/oder ein Winkel zwischen der Beaufschlagungsfläche 25 und der Verschiebungsrichtung 41 bleibt während einer Betätigung des zweiten Griffteils 4 unverändert. Lediglich der Kontaktpunkt 26 des Eingriffszapfens 24 mit der Beaufschlagungsfläche 25 und/oder die Ausrichtung des Eingriffszapfens 24 insbesondere innerhalb der Ausgleichsvorrichtung 23 kann sich während einer Betätigung des zweiten Griffteils 4 verändern. Der Eingriffszapfen 24 kann einen vorzugsweisen zylinderförmigen Kopfteil mit rundem Querschnitt aufweisen, über welchen Kopfteil der Eingriffszapfen 24 durch Beaufschlagung der Beaufschlagungsfläche 25 den Kontaktpunkt 26 mit dieser herstellt.

Um den Abstand zwischen den beiden Griffteilen 3 und 4 möglichst gering halten zu können, ist am ersten Griffteil 3 und am zweiten Griffteil 4 jeweils eine Eintauchausnehmung 27, 28 ausgebildet. In Schließstellung, also wenn ein Abstand zwischen dem ersten Griffteil 3 und dem zweiten Griffteil 4 am geringsten ist, tauchen die Führungselemente 10, 11 und/oder das Antriebselement 15 in die Eintauchausnehmungen 27, 28 am ersten Griffteil 3 und am zweiten Griffteil 4 ein.

Um das chirurgische Instrument 1 aus seiner Schließstellung zurück in seine Offenstellung, also in die Stellung, in welcher das erste Griffteil 3 und das zweite Griffteil 4 maximal voneinander entfernt sind, ohne Aufbringen einer Handkraft zurückstellen zu können, weist das chirurgische Instrument 1 ein Rückstellelement 29 auf. Eine Rückstellkraft des Rückstellelements 29 wirkt einer Schließbewegung des chirurgischen Instruments 1 entgegen.

Um eine besonders sichere Lagerung des Rückstellelements 29 zu erreichen, ist am ersten Führungselement 10 eine Rückstellelementaufnahme 30 ausgebildet, in welche das Rückstellelement 29 eingesetzt ist.

Durch das Rückstellelement 29 ist das erste Führungselement 10 gegen das erste Griffteil 3 abgestützt. Das Rückstellelement 29 kann, wie in den Figuren 1 bis 8 gezeigt ist, beispielsweise als eine Feder, insbesondere als eine Schenkelfeder 31 ausgebildet sein. Das Rotationszentrum der Schenkelfeder 31 kann dabei, wie in den Figuren 3 bis 8 gut zu erkennen ist, dem Gelenkpunkt 5 des ersten Führungselements 10 am ersten Griffteil 3 entsprechen oder mit diesem überlappen.

Das erste Führungselement 10 weist eine obere Anlagefläche 33 und eine untere Anlagefläche 34 auf. Das zweite Führungselement 11 weist eine obere Anlagefläche 35 und eine untere Anlagefläche 36 auf. In Offenstellung des chirurgischen Instruments 1 liegen die beiden Anlageflächen 34 und 36 aneinander an. In Schließstellung liegen die beiden Anlageflächen 33 und 35 aneinander an.

Der Gelenkpunkt 5 des Antriebselements 15 am ersten Griffteil 3 ist weiterentfernt von der Schaftaufnahme 12 und/oder dem Schaft 19 angeordnet als die Gelenkpunkte 5 der Führungselemente 10, 11 am ersten Griffteil 3. Somit ist der Hebel des Antriebselements 15, der auf die Ausgleichsvorrichtung 23 wirkt, möglichst lang ausgebildet, um einen möglichst großen Hub des chirurgischen Instruments 1 zu erreichen.

Das Antriebselement 15 ist wenigstens teilweise in einer Aussparung 37 am zweiten Führungselement 11 angeordnet. Somit flankiert das zweite Führungselement 11 durch seine geteilte Ausgestaltung mit jeweils einem Teil das Antriebselement 15 wenigstens teilweise lateral.

Ein freies Ende 39 des zweiten Griffteils 4 ist in Ruheposition in Richtung des ersten Griffteils 3 geneigt, so dass ein Betätigungsweg 9 für den in der Nähe oder am freien Ende 39 anzusetzenden kleinen Finger einer Hand möglichst klein gehalten ist.

Das freie Ende 39 des zweiten Griffteils 4 ist somit in einer Ruhestellung, also einer Offenstellung, näher am ersten Griffteil 3 gelegen als ein Griffmittelpunkt 40 des zweiten Griffteils 4. Der Griffmittelpunkt 40 ist etwa auf halber Strecke der Handanlagefläche 14 am zweiten Griffteil 4 angeordnet.

Am zweiten Führungselement 11 ist eine Drehachsenaussparung 42 ausgebildet, in welche die Drehachse des Lagerpunkts 16 des Antriebselements 15 in Schließstellung des chirurgischen Instruments 1 eingreift.

Das erste Griffteil 3 ist beispielsweise an einer Handanlagefläche 13 gebogen, also nicht geradlinig ausgebildet. Insbesondere weist das erste Griffteil 3 eine S-Form auf. An der Handanlagefläche 13 des ersten Griffteils 3 kann eine Handballenaussparung ausgebildet sein.

Das zweite Griffteil 4 ist beispielsweise an einer Handanlagefläche 14 gebogen ausgestaltet. Insbesondere weist das zweite Griffteil 4 eine C-Form oder eine S-Form auf.

Das hierin beschriebene und beanspruchte chirurgische Instrument eignet sich besonders zur Bewegung eines Schneide- und/oder Stanzwerkzeugs 22. Das Schneide- und/oder Stanzwerkzeug 22 kann dabei beispielsweise mit der Gleitschiene 21 verbunden sein, wie in den Figuren 1 bis 8 angedeutet ist.

Die Erfindung betrifft also insbesondere ein chirurgisches Instrument 1 mit einem Griffbereich 2, der ein in Gebrauchsstellung feststehendes erstes Griffteil 3 und ein relativ zum ersten Griffteil 3 entlang einer Verstellungsrichtung 6 bewegbares zweites Griffteil 4 aufweist, wobei ein Verhältnis zwischen einer ersten Strecke 7 zwischen dem ersten Griffteil 3 und dem zweiten Griffteil 4 und einer zweiten Strecke 8 zwischen dem ersten Griffteil 3 und dem zweiten Griffteil 4 während einer Bewegung des zweiten Griffteils 4 in Richtung des ersten Griffteils 3 wenigstens über einen Teilbereich des Betätigungsweges 9 konstant oder nahezu konstant ist.

### Bezugszeichenliste

- 1: chirurgisches Instrument
- 2: Griffbereich
- 3: erstes Griffteil
- 4: zweites Griffteil
- 5: Gelenkpunkt
- 6: Verstellungsrichtung
- 7: erste Strecke
- 8: zweite Strecke
- 9: Betätigungsweg
- 10: erstes Führungselement
- 11: zweites Führungselement
- 12: Schaftaufnahme
- 13: Handanlagefläche am ersten Griffteil
- 14: Handanlagefläche am zweiten Griffteil
- 15: Antriebselement
- 16: Lagerpunkt
- 17: Gelenkpunkt mit wandernder Drehachse
- 18: Biegung
- 19: Schaft
- 20: Führungsschiene
- 21: Gleitschiene
- 22: Schneid- und/oder Stanzwerkzeug
- 23: Ausgleichsvorrichtung
- 24: Eingriffszapfen
- 25: Beaufschlagungsfläche
- 26: Kontaktpunkt
- 27: Eintauchausnehmung am ersten Griffteil
- 28: Eintauchausnehmung am zweiten Griffteil
- 29: Rückstellelement
- 30: Rückstellelementaufnahme
- 31: Schenkelfeder
- 32: Rotationszentrum der Schenkelfeder
- 33: obere Anlagefläche des ersten Führungselements
- 34: untere Anlagefläche des ersten Führungselements
- 35: obere Anlagefläche des zweiten Führungselements
- 36: untere Anlagefläche des zweiten Führungselements
- 37: Aussparung am Führungselement
- 38: Führungsabschnitt; Langloch
- 39: freies Ende des zweiten Griffteils
- 40: Griffmittelpunkt des zweiten Griffteils
- 41: (geradlinige) Bewegung der Ausgleichsvorrichtung; Verschiebungsrichtung
- 42: Drehachsenaussparung

## Patentansprüche

1. Chirurgisches Instrument (1), insbesondere zur Gewebeabtrennung, mit einem Griffbereich (2), der ein in Gebrauchsstellung feststehendes erstes Griffteil (3) und ein relativ zum ersten Griffteil (3) in einer Verstellungsrichtung (6) bewegbares zweites Griffteil (4) aufweist, wobei am zweiten Griffteil (4) wenigstens zwei versetzt zueinander angeordnete Gelenkpunkte (5, 17) ausgebildet sind, durch welche die Verstellungsrichtung (6) des zweiten Griffteils (4) relativ zum ersten Griffteil (3) definiert ist, wobei die Verstellungsrichtung (6) des zweiten Griffteils (4) durch wenigstens zwei Führungselemente (10, 11) festgelegt ist, wobei jedes Führungselement (10, 11) jeweils über einen Gelenkpunkt (5) mit dem ersten Griffteil (3) und über jeweils einen Gelenkpunkt (5) mit dem zweiten Griffteil (4) verbunden ist, wobei deren Gelenkpunkte (5) am ersten Griffteil (3) versetzt zueinander angeordnet sind, und wobei deren Gelenkpunkte (5) am zweiten Griffteil (4) versetzt zueinander angeordnet sind, **dadurch gekennzeichnet, dass** das chirurgische Instrument (1) ein Antriebselement (15) aufweist, das über einen Lagerpunkt (5, 16, 17) mit dem ersten Griffteil (3) und über einen Lagerpunkt (5, 16, 17) mit dem zweiten Griffteil (4) verbunden ist.

2. Chirurgisches Instrument (1) nach Anspruch 1, **dadurch gekennzeichnet, dass** das Antriebselement (15) über eine wandernde Drehachse mit dem zweiten Griffteil (4) verbunden ist und/oder dass das Antriebselement (15) über eine wandernde Drehachse mit dem ersten Griffteil (3) verbunden ist, beispielsweise wobei der Lagerpunkt (5, 16, 17) oder die Lagerpunkte (5, 16, 17) des Antriebselements (15) am ersten Griffteil (3) und/oder am zweiten Griffteil (4) zu einem oder dem Gelenkpunkt (5) zumindest eines der Führungselemente (10, 11) am ersten Griffteil (3) und/oder am zweiten Griffteil (4) versetzt angeordnet ist/sind.

3. Chirurgisches Instrument (1) nach einem der vorstehenden Ansprüche, **dadurch gekennzeichnet, dass** wenigstens eines der Führungselemente (10, 11) und/oder ein oder das Antriebselement (15) eine Biegung (18) aufweist/aufweisen, insbesondere wobei das wenigstens eine Führungselement (10, 11) und/oder das Antriebselement (15) in Richtung des ersten Griffteils (3) und/oder des zweiten Griffteils (4) geneigt sind.

4. Chirurgisches Instrument (1) nach einem der vorstehenden Ansprüche, **dadurch gekennzeichnet, dass** das chirurgische Instrument (1) einen Schaft (19) mit einer Führungsschiene (20) und einer relativ zur Führungsschiene (20) verstellbaren Gleitschiene (21) aufweist, wobei bei einer Betätigung des zweiten Griffteils (4) eine Kraftübertragung von wenigstens einem Führungselement (10, 11) und/oder von einem oder dem Antriebselement (15) auf die Gleitschiene (21) des Schaftes (19) erfolgt.

5. Chirurgisches Instrument (1) nach einem der vorstehenden Ansprüche, **dadurch gekennzeichnet, dass** das chirurgisches Instrument (1), insbesondere an einer oder der Gleitschiene (21), eine Ausgleichsvorrichtung (23) aufweist, in welche ein Eingriffszapfen (24), vorzugsweise ein am Antriebselement (15) ausgebildeter Eingriffszapfen (24), eingreift und eine Beaufschlagungsfläche (25) der Ausgleichsvorrichtung (23) kontaktiert, wobei bei einer Betätigung des zweiten Griffteils (4) eine Kreisbewegung des Eingriffszapfens (24) in eine geradlinige Bewegung (41) der Ausgleichsvorrichtung (23), insbesondere der Gleitschiene (21) des Schaftes (19), umgesetzt wird, insbesondere indem ein Kontaktpunkt (26) des Eingriffszapfens (24) an der Beaufschlagungsfläche (25) in einer Richtung quer oder senkrecht zur geradlinigen Bewegung (41) entlang der Beaufschlagungsfläche (25) wandert.

6. Chirurgisches Instrument (1) nach Anspruch 5, **dadurch gekennzeichnet, dass** die Ausgleichsvorrichtung (23) fest, insbesondere in einem Öffnungswinkel unverstellbar, mit einer oder der Gleitschiene (21) verbunden ist, insbesondere derart, dass ein Winkel zwischen der Beaufschlagungsfläche (25) und der Gleitschiene (21) während einer Betätigung des chirurgischen Instruments (1) unverändert bleibt.

7. Chirurgisches Instrument (1) nach einem der vorstehenden Ansprüche, **dadurch gekennzeichnet, dass** am ersten Griffteil (3) und/oder am zweiten Griffteil (4) eine Eintauchausnehmung (27, 28) zum Eintauchen wenigstens eines Führungselements (10, 11) in das erste Griffteil (3) und/oder das zweite Griffteil (4) ausgebildet ist.

8. Chirurgisches Instrument (1) nach einem der vorstehenden Ansprüche, **dadurch gekennzeichnet, dass** das chirurgische Instrument (1) ein Rückstellelement (29) aufweist, dessen Rückstellkraft einer Relativbewegung des ersten Griffteils (3) hin zum zweiten Griffteil (4) entgegenwirkt, insbesondere wobei an einem der Führungselemente (10, 11) eine Rückstellelementaufnahme (30) ausgebildet ist, in welche das Rückstellelement (29) eingesetzt ist.

9. Chirurgisches Instrument (1) nach einem der vorstehenden Ansprüche, **dadurch gekennzeichnet, dass** an den Führungselementen (10, 11) jeweils zwei, insbesondere unterschiedliche Ausrichtungen aufweisende, Anlageflächen (33, 34, 35, 36) ausgebildet sind, wobei ein erstes Paar an Anlageflächen (34, 36) in einer maximalen Offenstellung aneinander anliegen und ein zweites Paar an Anlageflächen (33, 35) in einer maximalen Schließstellung aneinander anliegen.

10. Chirurgisches Instrument (1) nach einem der vorstehenden Ansprüche, **dadurch gekennzeichnet, dass** ein oder der Lagerpunkt (5, 16, 17) eines oder des Antriebselements (15) am ersten Griffteil (3) weiter entfernt von einer oder der Schaftaufnahme (12) und/oder von einem oder dem Schaft (19) angeordnet ist als die Gelenkpunkte (5, 17) der Führungselemente (10, 11) am ersten Griffteil (3).

11. Chirurgisches Instrument (1) nach einem der vorstehenden Ansprüche, **dadurch gekennzeichnet, dass** ein oder das Antriebselement (15) wenigstens teilweise in einer Aussparung (37) in einem Führungselement (10, 11) angeordnet ist.

12. Chirurgisches Instrument (1) nach einem der vorstehenden Ansprüche, **dadurch gekennzeichnet, dass** ein freies Ende (39) des zweiten Griffteils (4) in Ruheposition in Richtung des ersten Griffteils (3) geneigt ist und/oder dass ein oder das freie Ende (39) des zweiten Griffteils (4) in Ruhestellung näher am ersten Griffteil (3) liegt als ein Griffmittelpunkt (40) des zweiten Griffteils (4).

## Claims

1. Surgical instrument (1), in particular for tissue separation, comprising a grip region (2) which has a first grip part (3) that is fixed in a position of use, and a second grip part (4) which is movable in an adjustment direction (6) relative to the first grip part (3), wherein, on the second grip part (4), at least two articulation points (5, 17) are formed which are offset relative to each other and by which the adjustment direction (6) of the second grip part (4) relative to the first grip part (3) is defined, wherein the adjustment direction (6) of the second grip part (4) is determined by at least two guide elements (10, 11), wherein each guide element (10, 11) is connected to the first grip part (3) via a respective articulation point (5) and connected to the second grip part (4) via a respective articulation point (5), wherein the articulation points (5) on the first grip part (3) are arranged offset to each other, and wherein the articulation points (5) on the second grip part (4) are arranged offset to each other, **characterized in that** the surgical instrument (1) has a drive element (15) which is connected to the first grip part (3) via a bearing point (5, 16, 17) and connected to the second grip part (4) via a bearing point (5, 16, 17).

2. Surgical instrument (1) according to Claim 1, **characterized in that** the drive element (15) is connected to the second grip part (4) via a moving pivot axis, and/or **in that** the drive element (15) is connected to the first grip part (3) via a moving pivot axis, for example wherein the bearing point (5, 16, 17) or the bearing points (5, 16, 17) of the drive element (15) on the first grip part (3) and/or on the second grip part (4) is/are arranged offset in relation to a or the articulation point (5) of at least one of the guide elements (10, 11) on the first grip part (3) and/or on the second grip part (4) .

3. Surgical instrument (1) according to one of the preceding claims, **characterized in that** at least one of the guide elements (10, 11) and/or a or the drive element (15) has a curve (18), in particular wherein the at least one guide element (10, 11) and/or the drive element (15) are inclined in the direction of the first grip part (3) and/or second grip part (4).

4. Surgical instrument (1) according to one of the preceding claims, **characterized in that** the surgical instrument (1) has a shaft (19) with a guide rail (20) and with a slide rail (21) adjustable relative to the guide rail (20), wherein, when the second grip part (4) is actuated, force is transmitted from at least one guide element (10, 11) and/or from a or the drive element (15) to the slide rail (21) of the shaft (19).

5. Surgical instrument (1) according to one of the preceding claims, **characterized in that** the surgical instrument (1) has, in particular on a or the slide rail (21), a compensation device (23) in which an engagement pin (24), preferably an engagement pin (24) formed on the drive element (15), engages and contacts an impingement surface (25) of the compensation device (23), wherein, when the second grip part (4) is actuated, a circular movement of the engagement pin (24) is converted into a rectilinear movement (41) of the compensation device (23), in particular of the slide rail (21) of the shaft (19), in particular by a contact point (26) of the engagement pin (24) on the impingement surface (25) moving in a direction transverse or perpendicular to the rectilinear movement (41) along the impingement surface (25) .

6. Surgical instrument (1) according to Claim 5, **characterized in that** the compensation device (23) is connected to a or the slide rail (21) in a fixed manner, in particular non-adjustably in an opening angle, in particular in such a way that an angle between the impingement surface (25) and the slide rail (21) remains unchanged during an actuation of the surgical instrument (1) .

7. Surgical instrument (1) according to one of the preceding claims, **characterized in that** a plunge recess (27, 28) is formed on the first grip part (3) and/or on the second grip part (4), for plunging at least one guide element (10, 11) into the first grip part (3) and/or the second grip part (4).

8. Surgical instrument (1) according to one of the preceding claims, **characterized in that** the surgical instrument (1) has a resetting element (29), a resetting force of which counteracts a relative movement of the first grip part (3) towards the second grip part (4), in particular wherein the resetting element (29) is inserted into a resetting element receptacle (30) formed on one of the guide elements (10, 11).

9. Surgical instrument (1) according to one of the preceding claims, **characterized in that** two contact surfaces (33, 34, 35, 36), in particular having different orientations, are formed on the guide elements (10, 11), wherein a first pair of contact surfaces (34, 36) bear on each other in a maximum open position and a second pair of contact surfaces (33, 35) bear on each other in a maximum closed position.

10. Surgical instrument (1) according to one of the preceding claims, **characterized in that** a or the bearing point (5, 16, 17) of a or the drive element (15) on the first grip part (3) is arranged further away from a or the shaft receptacle (12) and/or from a or the shaft (19) than the articulation points (5, 17) of the guide elements (10, 11) on the first grip part (3).

11. Surgical instrument (1) according to one of the preceding claims, **characterized in that** a or the drive element (15) is arranged at least partially in a cutout (37) in a guide element (10, 11).

12. Surgical instrument (1) according to one of the preceding claims, **characterized in that** a free end (39) of the second grip part (4), in a rest position, is inclined in the direction of the first grip part (3), and/or **in that** a or the free end (39) of the second grip part (4), in a rest position, is closer to the first grip part (3) than is a grip centre point (40) of the second grip part (4).

## Revendications

1. Instrument chirurgical (1), en particulier pour la séparation des tissus, avec une zone de préhension (2) qui comporte une première partie formant poignée (3) fixe en position d'utilisation et une deuxième partie formant poignée (4) mobile par rapport à la première partie formant poignée (3) dans une position de réglage, au moins deux points d'articulation (5, 17) décalés entre eux, par lesquels la direction de réglage (6) de la deuxième partie formant poignée (4) par rapport à la première partie formant poignée (3) est définie, étant formés sur la deuxième partie formant poignée (4), la direction de réglage (6) de la deuxième partie formant poignée (4) étant déterminée par au moins deux éléments de guidage (10, 11), chaque élément de guidage (10, 11) étant relié à la première partie formant poignée (3) par un point d'articulation (5) et à la deuxième partie formant poignée (4) par un point d'articulation (5), les points d'articulation (5) sur la première partie formant poignée (3) étant décalés entre eux et les points d'articulation (5) sur la deuxième partie formant poignée (4) étant décalés entre eux, **caractérisé en ce que** cet instrument chirurgical (1) comporte un élément d'entraînement (15) qui est relié par un point de fixation (5, 16, 17) à la première partie formant poignée (3) et par un point de fixation (5, 16, 17) à la deuxième partie formant poignée (4).

2. Instrument chirurgical (1) selon la revendication 1, **caractérisé en ce que** l'élément d'entraînement (15) est relié par un axe de rotation errant à la deuxième partie formant poignée (4) et/ou que l'élément d'entraînement (15) est relié par un axe de rotation errant à la première partie formant poignée (3), par exemple le point de fixation (5, 16, 17) ou les points de fixation (5, 16, 17) de l'élément d'entraînement (15) sur la première partie formant poignée (3) et/ou sur la deuxième partie formant poignée (4) étant décalés par rapport à un ou au point d'articulation (5) d'au moins un des éléments de guidage (10, 11) sur la première partie formant poignée (3) et/ou sur la deuxième partie formant poignée (4).

3. Instrument chirurgical (1) selon une des revendications précédentes, **caractérisé en ce qu'**au moins un des éléments de guidage (10, 11) et/ou un ou l'élément d'entraînement (15) présente(nt) un coude (18), en particulier l'au moins un élément de guidage (10, 11) et/ou l'élément d'entraînement (15) étant incliné(s) en direction de la première partie formant poignée (3) et/ou de la deuxième partie formant poignée (4).

4. Instrument chirurgical (1) selon une des revendications précédentes, caractérisé en ce cet instrument chirurgical (1) comporte une tige (19) avec un rail de guidage (20) et une glissière (21) réglable par rapport au rail de guidage (20), une transmission de force d'au moins un élément de guidage (10, 11) et/ou d'un ou de l'élément d'entraînement (15) sur la glissière (21) de la tige (19) se produisant lors d'un actionnement de la deuxième partie formant poignée (4).

5. Instrument chirurgical (1) selon une des revendications précédentes, caractérisé en ce cet instrument chirurgical (1) comporte, en particulier au niveau d'une ou de la glissière (21), un dispositif d'équilibrage (23) dans lequel un tenon d'engagement (24), de préférence un tenon d'engagement (24) formé sur l'élément d'entraînement (15), s'engage et entre en contact avec une surface d'application (25) du dispositif d'équilibrage (23), un déplacement circulaire du tenon d'engagement (24) étant converti en un déplacement rectiligne (41) du dispositif d'équilibrage (23), en particulier de la glissière (21) de la tige (19), lors d'un actionnement de la deuxième partie formant poignée (4), en particulier un point de contact (26) du tenon d'engagement (24) sur la surface d'application (25) se déplaçant dans une direction transversale ou perpendiculaire au mouvement rectiligne (41) le long de la surface d'application (25).

6. Instrument chirurgical (1) selon la revendication 5, **caractérisé en ce que** le dispositif d'équilibrage (23) est relié de façon solidaire, en particulier selon un angle d'ouverture non réglable, à une ou la glissière (21), en particulier de telle sorte qu'un angle entre la surface d'application (25) et la glissière (21) reste inchangé pendant un actionnement de l'instrument chirurgical (1).

7. Instrument chirurgical (1) selon une des revendications précédentes, **caractérisé en ce que** sur la première partie formant poignée (3) et/ou sur la deuxième partie formant poignée (4) est formée une cavité d'insertion (27, 28) pour insérer au moins un élément de guidage (10, 11) dans la première partie formant poignée (3) et/ou la deuxième partie formant poignée (4).

8. Instrument chirurgical (1) selon une des revendications précédentes, **caractérisé en ce que** cet instrument chirurgical (1) comporte un élément de rappel (29) dont la force de rappel s'oppose à un déplacement relatif de la première partie formant poignée (3) vers la deuxième partie formant poignée (4), en particulier un réceptacle d'élément de rappel (30) dans lequel l'élément de rappel (29) est inséré étant formé sur un des éléments de guidage (10, 11).

9. Instrument chirurgical (1) selon une des revendications précédentes, **caractérisé en ce que** sur chacun des éléments de guidage (10, 11) sont constituées deux surfaces d'application (33, 34, 35, 36) présentant en particulier des orientations différentes, une première paire de surfaces d'application (34, 36) s'appliquant l'une contre l'autre dans une position d'ouverture maximale et une deuxième paire de surfaces d'application (33, 35) s'appliquant l'une contre l'autre dans une position de fermeture maximale.

10. Instrument chirurgical (1) selon une des revendications précédentes, **caractérisé en ce qu'**un ou le point de fixation (5, 16, 17) d'un ou de l'élément d'entraînement (15) est disposé sur la première partie formant poignée (3) et plus éloigné d'un ou du logement de tige (12) et/ou d'une ou de la tige (19) que les points d'articulation (5, 17) des éléments de guidage (10, 11) sur la première partie formant poignée (3).

11. Instrument chirurgical (1) selon une des revendications précédentes, **caractérisé en ce qu'**un ou l'élément d'entraînement (15) est disposé au moins partiellement dans un évidement (37) dans un élément de guidage (10, 11).

12. Instrument chirurgical (1) selon une des revendications précédentes, **caractérisé en ce qu'**en position de repos, une extrémité libre (39) de la deuxième partie formant poignée (4) est inclinée en direction de la première partie formant poignée (3) et/ou qu'en position de repos, une ou l'extrémité libre (39) de la deuxième partie formant poignée (4) est plus proche de la première partie formant poignée (3) qu'un centre de préhension (40) de la deuxième partie formant poignée (4).
